# EUROPEAN PATENT APPLICATION

(11) **EP 1 361 281 A1**
(43) Date of publication of application: **12.11.2003**
(21) Application number: 02716366.6
(22) Date of filing: 24.01.2002
(51) Int. Cl.: C12P 23/00, C09B 61/00

(54) **A PROCESS FOR PURIFYING CRUDE XANTHOPHYLLS**

(30) Priority: 26.01.2001 JP 2001018323
(71) Applicant: Fuji Chemical Industry Co., Ltd., Nakaniikawa-gun, Toyama 939-0397 (JP); Osaka-Shi, Osaka-shi, Osaka 530-0005 (JP)
(72) Inventor: SHIMADA, Yuji, Osaka-shi, Osaka 546-0044 (JP); NAGAO, Toshihiro, Osaka-shi, Osaka 544-0024 (JP); SUGIHARA, Akio, Itami-shi, Hyogo 664-0898 (JP); TOMINAGA, Yoshio, Osaka-shi, Osaka 555-0021 (JP); IKUSHIMA, Heiji, Fuji Chemical Industry, Co., Ltd., Nakaniikawa-gun, Toyama 939-0397 (JP); TANAKA, Nobukazu, Fuji Chemical Industry, Co., Ltd, Nakaniikawa-gun, Toyama 939-0397 (JP); FUKAMI, Tadashi, Fuji Chemical Industry, Co., Ltd., Nakaniikawa-gun, Toyama 939-0397 (JP); IKAI, Hisato, Fuji Chemical Industry, Co., Ltd., Nakaniikawa-gun, Toyama 939-0397 (JP)
(74) Representative: Albrecht, Thomas, Dr.
(86) International application number: JP0200520
(87) International publication number: WO02059341

(57) **Abstract**

There is disclosed herein a process for purifying a crude xanthophyll which is characterized by acting a lipase on the crude xanthophyll, which is obtained by extracting algae with a solvent, in the present of water under an acidic condition without adjusting the pH whereupon the contaminating neutral lipids (mono-, di- and triglycerides) are selectively hydrolyzed, subjecting the lipase-treated liquor to oil/water separation and removing free fatty acids from the oil layer fractioned thereby elevating the content of xanthophyll.

## Description

### Technical Field

The present invention relates to a process for purifying crude xanthophylls, and more particularly it relates to a process for purifying crude xanthophylls, which makes it possible to provide high quality of xanthophylls so that they may be widely used in the fields of a food, a medicine, quasi-drugs, a cosmetic, a cultivation and so on, by hydrolyzing fatty esters of glycerin, principal contaminant derived from the raw material for preparing xanthophylls, with a lypase and removing the resultant hydrolyzate thereby increasing the content of xanthophyll components efficiently and further removing particular odor derived from the raw material.

### Background Art

The xanthophylls, one kind of carotenoids are compounds containing one or more hydroxyl groups in the cyclic structure moiety of the molecular skeleton and are present in two or three forms of xanthophyll (free form), mono- and di-esters thereof depending on the number of hydroxyl group. Synthetic xanthophylls or natural ones derived from animal and vegetable are known. As astaxanthin, astaxanthin monoester and astaxanthin diester (hereinafter, referred to as "astaxanthins" collectively) being one of the representative xanthophylls, both the synthetic ones and natural ones derived from animal and vegetable have been commercially sold. Specifically, they may be obtained from an animal such as a crab, a shrimp, a krill and the like by extraction, and they may also be obtained by squeezing oil from algae, for example green algae such as Haematococcus, chlorella, Scenedesmus and the like; from yeasts such as Falfa rhodozyma and the like; or from microorganisms such as bacteria, alternatively by extraction from them through organic solvents such as acetone, ethylacetate, dichloroethane and the like or otherwise an edible oil. The astaxanthins have been expected for utilization as a natural coloring agent, an antioxidant, a health food, a cosmetic and a medicine because they convert into provitamin A after taken in the body and because they have remarkable antioxidant action (Eiji Yamashita; "Food and Development" vol. 27, No. 3, p 38-40 (1992)).

Various processes are reported for extracting xanthophylls from a vegetable, green algae, bacteria, cruschimata, etc. If specifically astaxanthins are explained taking as the example, the followings are: a process for extracting astaxanthins with an alkyl ester of a fatty acid (Japanese Patent Application Laid-Open No. Sho 58-88358), a process for extracting them with trichloromonofluoromethane (Japanese Patent Application Laid-Open No. Hei 2-255711), a process wherein a shell of a shrimp or a crab is dipped in hydrochloric acid to remove calcium present in the shell and then the resultant shell is dipped in a sodium hydroxide solution for a neutral treatment and removal of the protein present in the shell and subsequently the resultant shell is dipped in a solvent comprising 80 % of alcohol for brewing and 20 % of water at a temperature of 78 ± 5 °C to separate and extract astaxanthin from the solvent (Japanese Patent Application Laid-Open No. Hei 9-301950), etc. Also, in case of yeasts, there is reported a process wherein the cell walls of yeast after cultivation are broken physically or by acting a cell wall decomposing enzyme thereon and then astaxanthin is extracted from the ruptured cell walls with a mixed solution of hexane and ethanol (Japanese Patent Application Laid-Open No. Hei 7-41687), or a process wherein the cell walls of Falfa rhodozyma yeast are ruptured by heat-treatment at a temperature of 40 °C or more (Japanese Patent Application Laid-Open No. Hei 8-214870). Also, as processes for extracting astaxanthins from algae, for example a process wherein Haematococcus is subjected to a physical rupturing treatment and thereafter astaxanthins are extracted (WO 89/06910), a process wherein the cell walls are ruptured by the application of turbulent flow under high pressure and the ruptured cell walls are dried and astaxanthins are extracted with an organic solvent (Japanese Patent Application Laid-Open No. Hei 9-111139) or a process wherein algae containing astaxanthins for example Haematococcus is treated with acetone heated (to 40 °C-boiling point) and then treated with a cell wall decomposing enzyme which comprises the respective components of β -1,3-glucanase, β -1,4-glucanase and β -glucuronidase to extract astaxanthins (Japanese Patent Application Laid-Open No. Hei 11-56346) is reported. In addition, a process for extracting astaxanthins from krill crust in a supercritical state (carbon dioxide) (Japanese Patent Application Laid-Open No. Hei 6-200179) or a process for extracting them from bacteria is reported.

However, since the product obtained by the various processes as stated above contains contaminants (mainly fatty esters of glycerin) derived from the raw material, it is difficult to obtain highly concentrated pigment. Also, the obtained xanthophyll pigment has a particular odor derived from the raw material, and therefore it has a problem that its usefulness is limited.

In order to improve such faults, various separating and purifying processes have been proposed for obtaining highly concentrated xanthophylls free of particular odor. For example, the following processes are reported: ① a process for preparing yellowish orange or reddish orange pigment which comprises adjusting a pH of a krill extraction liquor to neutrality and decomposing fatty acids and other contaminants with a lipase or an alkali thereby forming a liquid system and subjecting the formed liquid system to a supercritical gas extraction or to a molecular distillation, or otherwise washing it with a dilute alkali (Japanese Patent Application Laid-Open No. Sho 60-4558), ② a process for preparing orange pigment wherein the unsaturated lipid other than the pigment present in the solvent extraction liquor of krill is hydrogenated and hydrolyzed with a lipase, and the liberated fatty acid is removed as urea adduct and/or by molecular distillation (Japanese Patent Publication No. 63-40827), ③ a process for preparing dark orange-red pigment wherein a solvent extract liquor of krill is purified by molecular distillation, column chromatography HPLC, etc. without subjecting it to chemical reactions such as hydrogenation and hydrolysis (Japanese Patent Application Laid-Open No. Hei 5-155736), ④ a process wherein a mineral acid is added to alkali-treated oleo-resin containing carotenoid pigment and the resultant carotenoid pigment is purified by molecular distillation (Japanese Patent Publication No. Sho 61-52184), ⑤ a process wherein a solution comprising an aqueous organic solvent containing xanthophylls is extracted with nonpolar solvent and water and thereafter the nonpolar solvent layer is concentrated or water is added thereto for extraction or nonpolar solvent is added for purification (Japanese Patent Application Laid-Open No. Hei 8-253695), etc. Among these prior art processes, however, the process employing a solvent extract liquor of krill as a raw material, for example the above ① process is one wherein water is added to a krill pigment solution obtained from the solvent extraction liquor of krill and the resultant solution is adjusted to neutrality (pH 7.0) and thereafter a lipase is added thereto to decompose fatty acid and other contaminants and to make a liquid system, which is subjected to a supercritical gas extraction or to molecular distillation or otherwise which is washed with a dilute alkali. According to this process, the content of pigment is reported to increase after the lipase treatment. But this is assumed to be due to the liberation of the pigment from one bonded with protein present in the krill extract.

There has been desired the development of a process which makes it possible to purify astaxanthin (free form), its monoester, diester, etc. stably and efficiently without changing the composition ratio of each astaxanthin component during separation and purification treatments.

An object of the present invention is to obtain high quality of concentrated and purified xanthophyll which may be widely used in the fields of a food, a medicine, quasi-drugs, a cosmetic, a cultivation and so on, by using a crude xanthophyll which is obtained by extracting f algae with solvent and removing the contaminants and peculiar odor derived from the raw material efficiently, unlike the processes using a solvent extraction liquor of krill.

### Disclosure of the Invention

Considering these circumstances, the present inventors have found that by using as the raw material a crude xanthophyll which is being produced in large amount by culturing Haematococcus alga, extracting it with organic solvents such as acetone, ethyl acetate, dichloroethane and the like and acting a lipase on the crude xanthophyll under a mild condition, the contaminating glycerin esters (mono-, di- and triglycerides) may be selectively hydrolyzed without entirely affecting xanthophyll esters, and that the lipase-treated liquor is allowed to settle for oil/water separation and the oil layer is distilled, thereby xanthophylls may be concentrated and purified efficiently with maintaining the content of their components present in the raw material quantitatively without changing the component ratio, and have completed the present invention.

That is, the invention involving in claim 1 is a process for purifying for elevating the content of xanthophyll which comprises acting a lipase on a crude xanthophyll, which is obtained by extracting algae with a solvent, in the present of water under an acidic condition without adjusting the pH whereupon the contaminating neutral lipids (mono-, di- and triglycerides) are selectively hydrolyzed, subjecting the lipase-treated liquor to oil/water separation and removing free fatty acids from the oil layer fractioned, the invention involving in claim 2 is a process for purifying a crude xanthophyll as claimed in claim 1 wherein the crude xanthophyll which is obtained by extracting algae with a solvent is crude astaxanthin, crude zeaxanthin, crude lutein, crude tunaxanthin, crude fucoxanthin, crude violaxanthin, crude neoxanthin or crude capthaxanthin, the invention involving in claim 3 is a process for purifying a crude xanthophyll as claimed in claim 1 wherein the crude xanthophyll is a crude astaxanthin and an acetone extract of Haematococcus algae and the invention involving in claim 4 is a process for purifying a crude xanthophyll as claimed in claim 1 wherein the lipase is one belonging to genus *Candida.*

### Brief Explanation of the Drawing

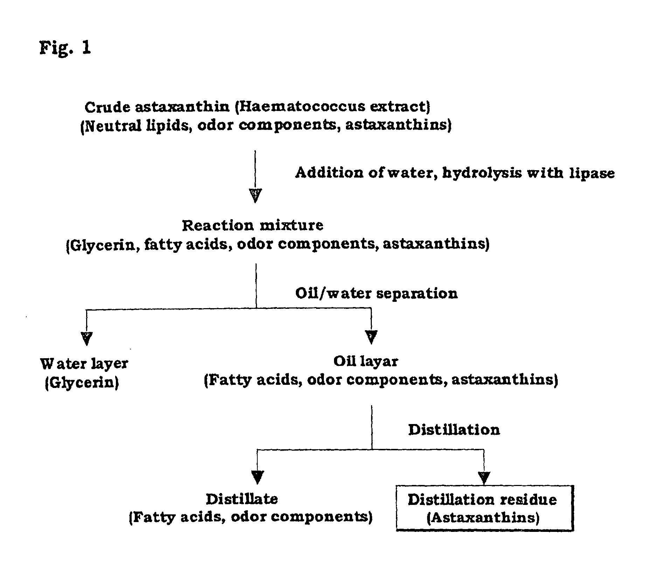

Fig. 1 is a flow chart in case where the present process was applied to the crude astaxanthin extracted from Haematococcus.

The followings illustrate the present invention in detail.

The xanthophylls are compounds containing one or two hydroxyl groups in the cyclic structure moiety of the molecular skeleton. Specifically, astaxanthin, zeaxanthin, lutein, tunaxanthin, fucoxanthin, violaxanthin, neoxanthin, capthaxanthin and the like may be taken.

The above-described xanthophylls are present in the forms of monoester and further diester depending on the number of hydroxyl group in addition to the free form.

The crude xanthophylls in the present invention are ones which may be obtained by a method wherein algae, for example Haematococcus alga is ruptured and extracted with suitable organic solvents such as acetone, ethyl acetate, dichloroethane and the like, and their esters, which are hydrolyzed with a lipase, for example mono-, di- and triglycerides which are glycerin esters are present in admixture as the contaminant.

Since the above-described crude xanthophylls are different in the content of xanthophylls, the compound ratio of each constituting component thereof, the kind, the content of the coexisting contaminants thereof and the compound ratio of each constituting component thereof depending on the kind and the gathering time (season) of algae to be extracted, the extraction technique, the purification and treatment method, etc., they are not limited particularly. However, a commercial product, for example "Algasunred" (a trade name, a product of Microbio Resources Inc.) which is commercially produced and easily available as being Haematococcus containing astaxanthin at high concentration is reported that with respect to the constituent components, they are 10-20 % of a protein, 10-20 % of a fat, 20-60% of a carbohydrate, 10-30 % of ash, 2-10 % of water and 1.5 % of the total xanthophyll (80 % or more of astaxanthin is contained in the total xanthophylls) and 10-20 % of a fat shown in the above is present in the forms of mono-, di- and triglycerides which are glycerin esters.

The crude xanthophylls which may be employed as the raw material in the present invention may be extracted from algae. As algae, for example green algae belonging to genus Haematococcus which produce astaxanthin may be employed. Specifically, *Haematococcus pluvialis, Haematococcus lacustris, Haematococcus capensis, Haematococcus droebakensis, Haematococcus zimbabwiensis,* etc., more specifically, green algae such as *Haematococcus pluvialis* NIES 144, *Haematococcus lacustris* ATCC 30402, ATCC 30453, IAM C296, IAM 392, IAM 393, IAM 394, IAM 399, *Haematococcus capensis* UTEX 1023, *Haematococcus droebakensis* UTEX 55, *Haematococcus zimbabwiensis* UTEX 1758, etc. may be taken.

Haematococcus alga may be cultivated according to the known method [for example, Kobayashi M. et al: J. Ferment. Bioeng., 74, 17-20 (1992)]. The nutrient medium which may be used for culturing Haematococcus alga may be any one, if green algae are grown therein. Examples of a carbon source include pyruvic acid, ethanol, ATC related organic acid and the like in addition to acetic acid which has been hitherto known. As a nitrogen source, an organic or inorganic nitrogen substance may be used. For example, amino acids such as asparagine, glycine, glutamic acid and the like; yeast extract, nitrite and so on may be taken. Not only the culture medium containing these carbon and nitrogen sources in combination is used, but also an inorganic salt may be added thereto depending on the necessity.

In order to enhance the synthesis of astaxanthin which is a vegetative cell for Haematococcus alga for obtaining the starting material for use in the present invention, there may be Haematococcus alga subjected to various stresses whereby the synthesis of astaxanthin has been induced and activated. As the means for inducing and activating the synthesis of astaxanthin as stated above, stresses such as nutrient starvation, a strong light, an active oxygen, an elevated temperature, a drying and so on may be imposed on Haematococcus alga singly or in combination thereby the nutrition cell may be converted into cyst cell in dormant state rapidly. In the present invention, there may also be the raw material extracted from the alga in which thus astaxanthin accumulates in large amount.

The cyst cell of Haematococcus alga cultivated thus may be easily recovered by centrifugal separation and so on. After the cell walls have been subjected to the rupturing treatment, an extraction process of astaxanthin is conducted according to the conventional method. For example, as a process for extracting the Haematococcus alga with an organic solvent, a process wherein cyst cell is dipped in an acetone solution for the prescribed time is known. For extraction, the solvent is removed by centrifugal separation and so on and thereafter the cell wall decomposing enzyme or drying treatment (e.g. freeze drying or spray drying) is conducted thereby the extraction efficiency may be further enhanced.

The extract derived from the above Haematococcus comprises the free form, monoester and diester as astaxanthins. Also, it comprises the free form, monoester and diester as the stereoisomers (3R, 3R'), (3R, 3'S) and (3S, 3'S). The process of the present invention may be applied efficiently even they are present in any compound ratio.

The pH of a suspension in water of the crude xanthophyll which may be obtained by the process described above is acidic. Although the pH value may be varied depending on the amount used of xanthophylls, the amount of water and so on, usually it is less than pH 7 owing to the coexistence of free fatty acids. In considering the reaction efficiency and economy, the preferable pH range is pH 2-6, more preferable one is pH 2-4.

A lipase may be any one irrespective of the origin, if it is used in the enzymatic reaction. Examples of a lipase include lipase derived from genus *Candida* microorganism e.g. genus *Candida (Candida rugosa,* a trade name "Lipase-OF", a product of Meito Sangyo Co., Ltd.), lipase derived from genus *Candida (Candida antarctica)* (a trade name "lipase Novozym 435, a product of Novozymes), lipase derived from genus *Chromobacterium* microorganism (lipase derived from *Chromobacterium viscosum*, a trade name "Lipase AC", a product of Asahi Chemical Industry Co., Ltd.), lipase derived from animal pancreas (e.g. a commercial product derived from pig pancreas produced by Aldrich), one derived from filamentous fungi *(Aspergillus niger),* or ), one derived from genus *Pseudomonas* lipase, further ones derived from genus *Geotrichum (Geotrichum candidum,* Osaka Municipal Technical Research Institute), filamentous fungi *(Aspergillus niger,* a trade name "Lipase AP", a product of Amano Pharmaceutical Co., Ltd.), genus *Pseudomonas (Pseudomonas glumae,* a trade name "Lipase-TOYO", a poduct of Asahi Chemical Industry Co., Ltd.), genus *Pseudomonas (Pseudomonas cepacia,* a trade name "Lipase-SL", a product of Meito Sangyo Co., Ltd.), Alcaligenes *sp*. (a trade name "Lipase-QLM", a product of Meito Sangyo Co., Ltd.), genus *Pseudomonas (Pseudomonas sp*. , a trade name "Lipase-PS", a product of Amano Pharmaceutical Co., Ltd.), genus *Rhizopus (Rhizopus delemar,* a trade name "TA-lipase", a product of Tanabe Seiyaku Co., Ltd.), genus *Pseudomonas (Pseudomonas sp. , a* trade name "Lipase-AK", a product of Amano Pharmaceutical Co., Ltd.), genus *Pseudomonas (Pseudomonas aeruginosa,* a trade name "LPL", a product of Toyobo Co., Ltd.), genus *Pseudomonas (PSEUDOMONAS sp. ,* a trade name "LIPOSAM", a product of Showa Denko K.K.), genus *Pseudomonas (PSEUDOMONAS stutzeri,* a trade name "Lipase-TL", a product of Meito Sangyo Co., Ltd.), genus *Rhizomucor miehei (Rhizomucor miehei,* a product of Novozymes), genus *Serratia (Serratia),* genus *Penicillium (Penicillium),* genus *Bacillus (Bacillus),* genus *Humicola lanuginose (Humicola lanuginose,* a product of Novo Nordisk) and so on.

Among the above described lipases in the present invention, preferable are *Candida rugosa* (a trade name "Lipase-OF", a product of Meito Sangyo Co., Ltd.), *Geotrichum candidum* (Osaka Municipal Technical Research Institute), *Aspergillus niger* (a trade name "Lipase AP", a product of Amano Pharmaceutical Co., Ltd.), *PSEUDOMONAS glumae* (a trade name "Lipase-TOYO", a poduct of Asahi Chemical Industry Co., Ltd.), *PSEUDOMONAS cepacia* (a trade name "Lipase-SL", a product of Meito Sangyo Co., Ltd.), *Alcaligenes sp.* (a trade name "Lipase-QLM", a product of Meito Sangyo Co., Ltd.), *PSEUDOMONAS sp.* (a trade name "Lipase-PS", a product of Amano Pharmaceutical Co., Ltd.), *Rhizopus delemar* (a trade name "TA-lipase", a product of Tanabe Seiyaku Co., Ltd.) and *PSEUDOMONAS sp.* (a trade name "Lipase-AK", a product of Amano Pharmaceutical Co., Ltd.), more preferable are *Candida rugosa* (a trade name "Lipase-OF", a product of Meito Sangyo Co., Ltd.), *Geotrichum candidum* (Osaka Municipal Technical Research Institute), *Aspergillus niger* (a trade name "Lipase AP", a product of Amano Pharmaceutical Co., Ltd.), *PSEUDOMONAS glumae* (a trade name "Lipase-TOYO", a poduct of Asahi Chemical Industry Co., Ltd.), *PSEUDOMONAS cepacia* (a trade name "Lipase-SL", a product of Meito Sangyo Co., Ltd.), *PSEUDOMONAS sp.* (a trade name "Lipase-PS", a product of Amano Pharmaceutical Co., Ltd.), and *Rhizopus delemar* (a trade name "TA-lipase", a product of Tanabe Seiyaku Co., Ltd.).

These lipases may be used singly or in combination of one or more kinds in the present invention.

The above described lipases may be used as powder or in the form fixed on carriers such as silica gel, celite, K -carrageenan, chitin, sodium alginate, an ion exchange resin, a porous carrier, ceramics, membrane and so on according to the conventional method.

Although the amount of lipase for use in the present invention is not particularly limited in the present invention, it may be 0.1 U (=unit)/g-10,000 U/g, preferably 1-5,000 U/g, more preferably 5-5,000 U/g. When it is less than 0.1 U/g, hydrolysis velocity becomes slow. Also, when it is greater than 5,000 U/g, the effect is sufficient but it does not become great in relative to the amount added of lipase and in some cases it is not preferable from the economical aspect.

One U of lipase means the amount of enzyme with which I mol of free fatty acid liberates in a minute in the hydrolysis of olive oil.

In order to hydrolyze the contaminating mono-, di- and triglycerides selectively, although the reaction temperature may be varied depending on the condition such as the optimum temperature and optimum pH of enzyme toward each of the contaminant components and it is not particularly limited, it is usually 4-90 °C, preferably the range of 10-70 °C. When the reaction temperature is higher than 70 °C, since the deterioration of astaxanthins present in the raw material promotes it is not preferable. Contrary thereto, when the reaction temperature is lower than 4 °C, since the activity of lipase decreases so that the reaction velocity becomes slow, it is not preferable. The pH is not necessary to be adjusted particularly and it may be an acidic condition which xanthophylls themselves show.

In the selective hydrolysis, a very amount of an organic solvent such as n-hexane, acetone, ethanol, methanol or the like which has been used for extraction may be present in the reaction system.

Since the reaction time may vary depending on the kind of enzyme to be used, the kind, composition and amount used of the raw material or the reaction temperature, it is not limited particularly. However, it is usually 0.5-150 hours, preferably 1-150 hours, more preferably 5-72 hours.

As the reaction mode, operation of settlement, stirring, shaking or the like may be applied. However, the reaction proceeds efficiently by increasing the dispersion state of the reaction,

In order to inhibit the deterioration reaction by oxidation of xanthophylls, the enzymatic reaction may be conducted under the atmosphere of an inert gas such as nitrogen, argon or the like. Also, an antioxidant may be added to the reaction system depending on the necessity. As the antioxidant, specifically tocophenol, tocotorienol, vitamin C and so on can be taken.

Although the amount of water for use in the hydrolysis is not limited particularly, it is usually 0.1-99 % by weight, preferably 5-90 % by weight based on the amount of the crude xanthophylls to be added to the reaction system.

When the amount of water is too small, the hydrolysis velocity is slow while when it is too great, the reaction liquor becomes dilute so that the use of a great quantity of a reaction vessel are required.

In the present invention, it is recommendable that the amount of water during the reaction is greater than 0.1 % by weight. When it is less than 0.1 % by weight, since the esterification reaction or ester exchange reaction occurs it is not preferable.

By the enzymetic reaction, the contaminants, particularly mono-, di- and triglycerides may be hydrolyzed to convert into glycerin and fatty acids. At this time, the lipase scarcely acts on xanthophyll ester form and hence the composition of xanthophylls prior to the reaction does not change. After completion of the enzymatic reaction, the reaction liquor is allowed to settle thereby two layers of oil and water layers is separated and glycerin migrates into the water layer. When the oil layer is fractioned by a distillation, fatty acids migrate into the distillate while xanthophylls migrate into the residue. Also, molecular distillation technique may be preferably applied as the distillation technique. By carrying out molecular distillation, particular odor components may be removed and therefore the residual xanthophylls fractions become no longer odor.

### The Best Mode For Carrying Out The Invention

The present invention is illustrated specifically based on the following Referential Examples and Examples but it does not restricted to these Examples.

Incidentally, the crude astaxanthin used in the following is one obtained from Haematococcus green alga cultured according to the known method by extracting with acetone and removing the extraction solvent under reduced pressure according to the conventional method.

The reaction status in each step involving in the present invention was confirmed by an acid value, HPLC, absorbance, TLC, etc.
(1) Acid value (mg KOH/g) is shown in weight amount (mg number) of potassium hydroxide necessary to neutralize fatty acids contained in 1 g of sample.
(2) HPLC method
   The solution used in the measurement of absorbance was passed through a filter (0.5 µm) to make a sample for HPLC. Each ratio of free form, monoester and diester of astaxanthin were calculated from the corresponding peak area value developed under the following HPLC ["JASCO 1500 System" a product of JASCO Corporation] condition.
   HPLC condition
   Moving bed: n-hexane (special class, a product of Wako Pure Chemical Industries, Ltd.) : acetone = 35:65
   Column: Develosil Packed Column (4.6 × 250 mm), Develosil300 DIOL-5
   Flow velocity: 0.5 ml/min.
   Detector wavelength: 480 nm
   Injected amount: 10 µL
   Time: 10 min.
(3) The content of astaxanthin in each sample was determined by absorptiometry (wavelength: 475 n, solvent: acetone). Incidentally, a commercial astaxanthin [a trade name, "ASTAXANTHIN", a product of Sigma Corporation] was used as a standard sample.

TLC was developed by hexane-ethyl acetate-acetic acid (80:20:1) using a silica gel plate. Sulfuric acid/methanol (1:1 vol/vol) was sprayed thereon and coloration was caused under heating according to the conventional manner.

### Referential Example 1

A ruptured and dried Haematococcus alga (a trade name "naturose", a product of Cyanotech Corporation) which contains astaxanthins and which is obtained by cultivation according to the conventional method was extracted with acetone and the acetone extract was concentrated with an evaporator to obtain a concentrate.

### Referential Example 2

"Crude astaxanthin" used in the following Examples means one obtained by removing solvent sufficiently under reduced pressure from the concentrate obtained in Referential Example 1. The astaxanthins contained in this crude astaxanthin were determined by the above stated absorptiometry and calculated in terms of the free form. The content of astaxanthin free form was 9.89 % by weight.

### Example 1

A variety of lipases were acted on the crude astaxanthin by the method as stated below.

First, 2 g of crude astaxanthin, 2 g of water and 200 units of each of a variety of lipases (50 units per g of the reaction mixture) shown in the following table 1 were added to a 50 ml of vial, respectively and then the prepared reaction liquor (a pH of 3.1 at this time) was incubated at 30 °C for 16 hours under stirring (at 500 rpm). After the reaction, an acid value in the oil layer fractioned by water/oil separation was measured.

The results are shown in table 1.

**Table 1**

| Kind of enzyme | Trade name | Acid value Acid value (mg KOH/g) |
|---|---|---|
| No addition | ― | 49.8 |
| *Candida rugosa* | Lipase―OF Meito Sangyo | 108.5 |
| *Geotrichum candidum* | Osaka Municipal Technical Research Institute | 89.9 |
| *Aspergillus niger* | Lipase―AP Amano Pharmaceutical | 85.5 |
| *Pseudomonas glumae* | Lipase―TOYO Asahi Chemical Industry | 81.8 |
| *Beudomonas capacia* | Lipase―SL Meito Sangyo | 79.6 |
| *Alcaligenes sp.* | Lipase --QLM Meito Sangyo | 73.0 |
| *Pseudomonas sp.* | Lipase ―PS Amano pharmaceutical | 70.7 |
| *Rhizopus delemar* | TA-lipase Tanabe Seiyaku | 62.2 |
| *Pseudomonas sp.* | Lipase ― OF Amano Pharmaceutical | 56.4 |

The crude astaxanthin contains neutral lipids as the contaminant. On hydrolysis of this neutral lipids fatty acids are formed and correspondingly the acid value representing the amount of free fatty acids elevates. It can be seen from the above results that since the action of a lipase causes increase in the acid value, the neutral lipids may be hydrolyzed efficiently. Among these lipases, especially *Candida rugosa* lipase made the neutral lipids be hydrolyzed the most efficiently.

### Example 2

The influence of the amount of enzyme on the hydrolysis of crude astaxanthin with *Candida rugosa* lipase (a trade name "Lipase-OF", a product of Meito Sangyo Co., Ltd.) " was examined.

The reaction liquor (a pH of 3.1-3.2 at this time) comprising 2 g of crude astaxanthin, 2 g of water and various amount of lipase was incubated at 30 °C for 16 hours under stirring (at 500 rpm) in a 50 ml vial. After the reaction (a pH of 2.9-3.1 at this time), the acid value in the oil layer fractioned by water/oil separation was measured.

The results are shown in table 2.

**Table 2**

| Amount of enzyme per gram Of the reaction mixture | Acid value (mg KOH/g) |
|---|---|
| 0 | 49.8 |
| 2.5 | 59.6 |
| 5 | 72.6 |
| 10 | 83.8 |
| 25 | 97.5 |
| 50 | 110.9 |
| 100 | 121.5 |
| 200 | 127.3 |

It can be seen from the above results that as the amount of enzyme is greater, the hydrolysis proceeds more efficiently.

### Example 3

The influence of the content of water on the hydrolysis of crude astaxanthin with *Candida rugosa* lipase (the same as that in Example 2) was examined.

The reaction mixture (a pH of 2.9-3.2 at this time) comprising 4 g of crude astaxanthin combined with of water and 200 units of lipase (50 units per gram of the reaction mixture) was incubated at 30 °C for 16 hours under stirring (at 500 rpm) in a 50 ml vial. After the reaction (a pH of 2.8-3.1 at this time), the acid value in the oil layer fractioned by water/oil separation was measured.

The results are shown in table 3.

**Table 3**

| Water content (%) | Acid value (mg KOH/g) |
|---|---|
| 75 | 120.8 |
| 50 | 110.9 |
| 25 | 99.9 |
| 10 | 87.4 |
| 5 | 59.3 |
| 0.3 | 48.1 |

From the above results, as the water content is larger, the efficiency of the hydrolysis is better.

### Example 4

A change with the progress of time in the hydrolysis of crude astaxanthin with *Candida rugosa* lipase (the same as that in Example 2) was examined.

The reaction mixture (a pH of 3.1 at this time) consisting of 2 g of crude astaxanthin, 2 g of water and 200 units of lipase (50 units per gram of the reaction mixture) was incubated at 30 °C under stirring (at 500 rpm) in a 50 ml vial. After the lapse of a time given, the acid value in the oil layer fractioned by water/oil separation was measured.

The results are shown in table 4.

**Table 4**

| Time (h) | Acid value (mgKOH/g) |
|---|---|
| 0 | 48.2 |
| 1 | 66.6 |
| 2 | 79.7 |
| 4 | 89.7 |
| 7 | 93.7 |
| 10 | 100.9 |
| 14 | 109.7 |
| 18 | 113.9 |
| 24 | 114.2 |
| 38 | 118.6 |
| 48 | 121.2 |
| 62 | 124.3 |
| 72 | 123.1 |

From the above results, the hydrolysis proceeds rapidly until about 14 hours from the beginning of the reaction and the reaction reaches almost equilibrium in 48 hours under the above reaction condition.

### Example 5: A large amount treatment of crude astaxanthin

600 Grams of crude astaxanthin (an acid value of 50.3), 400 ml of water and 200 units of *Candida rugosa* lipase (the same as that in Example 2) lipase (83 units per gram of the reaction mixture) was mixed together (a pH of 3.1 at this time) and allowed to react at 30 °C for 44 hours under stirring. After the reaction (a pH of 3.0 at this time), 588 g of the oil layer was recovered by water/oil separation. The acid value in this time was 88.0. After the filtration with celite, 54.0 g of a medium chain fatty acid triglyceride (MCT 85, caprylic acid 85 %, capric acid 15 %) was added in order to decrease the viscosity after the distillation (the weight amount at this time: 593.4 g). After the dehydration was carried out under reduced pressure (5 mmHg, 85 °C), 553.0 g of a sample having an acid value of 89.1 was obtained.

### (Molecular distillation)

The dehydrated sample was distilled at 160 °C under a degree of vacuum of 0.2 mmHg using thin film molecular distillator. The obtained residue was twice distillated at 180 °C and further the obtained residue was distillated at 200 °C. The weight amount and the acid value of the distillate and the residue obtained in each distillation step are shown in table 5.

**Table 5**

| | Sample | Weight (g) | Acid value (mg KOH/g) |
|---|---|---|---|
| 1st Distillation | 1st distillate | 77 | 197 |
| | 1st residue | 464 | 72 |
| 2nd Distillation | 2nd distillate | 156 | 172 |
| | 2nd residue | 299 | 15 |
| 3rd Distillation | 3rd distillate | 22 | 105 |
| | 3rd residue | 270 | 5.7 |

### (Determination of astaxanthin)

The content of astaxanthin determined by an absorptiometry and HPLC method is shown below.

The results of determination by absorptiometry are shown in table 6.

**Table 6**

| Sample Sample | Content of astaxanthin (%) |
|---|---|
| Raw material | 9.89 |
| After dehydration | 9.42 |
| 1st distillate | 0.01 |
| 1st residue | 10.56 |
| 2nd distillate | Trace |
| 2nd residue | 16.76 |
| 3rd distillate | Trace |
| 3rd residue | 17.98 |

Each ratio (%) of the free, monoester and diester forms of astaxanthin estimated by HPLC method is shown in table 7.

**Table 7**

| 3rd residue Sample | Diester (%) | Monoester (%) | Free form (%) |
|---|---|---|---|
| Raw material | 11.82 | 83.75 | 4.43 |
| After dehydration | 12.02 | 83.87 | 4.10 |
| 1st distillate | 6.90 | 41.66 | 51.45 |
| 1st residue | 12.14 | 83.70 | 4.16 |
| 2nd distillate | 8.96 | 86.03 | 5.01 |
| 2nd residue | 12.23 | 83.73 | 4.04 |
| 3rd distillate | 34.35 | 63.91 | 1.74 |
| 3rd residue | 12.27 | 83.74 | 3.99 |

The content of astaxanthin could be concentrated up to about 1.7 times by the above steps. Also, it was confirmed that the composition ratio of diester, monoester and free form of astaxanthin were not changed by the above treatment.

Incidentally, the reason that the content of astaxanthin decreased in the process of from the raw material to the dehydration is due to MCT 85 added before the dehydration.

### (Twice enzyme treatment)

Since the hydrolysis did not proceed sufficiently under the above enzymatic reaction to leave some neutral lipids, the third residue obtained by distillation was further subjected to the enzyme treatment and distillation.

260 grams of the third residue (an acid value of 5.66), 260 ml of water and 1.6 ml of 100 mg/ml *Candida* lipase (50 units per gram of the reaction system) were mixed together and allowed to react at 30 °C for 22 hours under stirring. After the reaction, 270 g of the oil layer was recovered by water/oil separation. At this time the acid value was 84.8. After the filtration with celite, the weight amount became 254 g. After the dehydration was carried out under reduced pressure (5 mmHg, 85 °C), 235 g of a sample having an acid value of 97.1 was obtained.

### (Distillation―second time)

The dehydrated sample was distilled at 160 °C under a degree of vacuum of 0.2 mmHg using a distillation apparatus. The obtained residue was twice distillated at 180 °C and further the obtained residue was distillated at 200 °C. The weight amount and the acid value of the distillate and the residue obtained in each distillation step are shown in table 8.

**Table 8**

| | Sample Sample | Weight (g) | Acid value (mg KOH/g) |
|---|---|---|---|
| 1st Distillation | 1st distillate | 45 | 269 |
| | 1st residue | 178 | 55 |
| 2nd Distillation | 2nd distillate | 25 | 208 |
| | 2nd residue | 149 | 29 |
| 3rd Distillation | 3rd distillate | 14 | 173 |
| | 3rd residue | 132 | 11.2 |

The content of astaxanthin determined by absorptiometry is shown in table 9.

**Table 9**

| Sample Sample | Content of astaxanthin Content (%) |
|---|---|
| Raw material | 17.98 |
| After dehydration | 17.64 |
| 1st distillate | 0.00 |
| 1st residue | 22.44 |
| 2nd distillate | 0.00 |
| 2nd residue | 26.13 |
| 3rd distillate | 0.00 |
| 3rd residue | 28.33 |

Also, each ratio (%) of the free, monoester and diester forms of astaxanthin estimated by HPLC method is shown in table 10.

**Table 10**

| Sample | Diester (%) | Monoester (%) | Free form (%) |
|---|---|---|---|
| Raw material | 12.27 | 83.74 | 3.99 |
| After dehydration | 11.96 | 82.07 | 5.97 |
| 1st distillate | ― | ― | ― |
| 1st residue | 12.18 | 83.38 | 4.44 |
| 2nd distillate | ― | ― | ― |
| 2nd residue | 12.14 | 83.19 | 4.67 |
| 3rd distillate | ― | ― | ― |
| 3rd residue | 12.32 | 83.17 | 4.51 |

The content of astaxanthin could be concentrated to about 1.5 time by the second enzymatic reaction and molecular distillation By combination of the first and second enzymatic reactions and distillations, the content of astaxanthin could be further concentrated to about 2.7 times. Incidentally, the composition ratio of diester, monoester and free form of astaxanthin was not changed during this treatment.

In the above treatment, MCT 85 was added in order to reduce the viscosity of the distillation residue in case of the first enzymatic reaction and distillation while it was not added in case of the second enzymatic reaction and distillation. Although molecular distillation may be conducted without adding this MCT 85, in case where MCT 85 was added the viscosity of the distillation residue is lowered so that the distillation procedure tends to be easily conducted.

It is recommendable that an organic solvent such as acetone present in the raw material be sufficiently removed in advance. When acetone is contained, since the enzymatic reaction does not proceed sufficiently the enzymatic reaction and distillation must be repeated in order to hydrolyze the impurities sufficiently. By using the raw material from which the solvent has been sufficiently removed, the rate of hydrolysis increases and sufficient concentration rate may be achieved by one time of the enzymatic reaction and distillation.

### Example 6

600 Grams of the crude astaxanthin (an acid value of 48.2), 600 ml of water and *Candida* lipase (50 units per gram of the reaction mixture) were mixed and allowed to react at 30 °C for 40 hours under stirring. After the reaction, 585 g of the oil layer was recovered by water/oil separation. At this time the acid value was 120. After the filtration with celite, the weight amount became 539 g. After the dehydration was carried out under reduced pressure (5 mmHg, 85 °C), 530 g of a sample having an acid value of 126 was obtained.

### (Distillation)

The dehydrated sample was distilled at 160 °C under a degree of vacuum of 0.2 mmHg using a distillation apparatus. The obtained residue was twice distillated at 180 °C and further the obtained residue was distillated at 200 °C. The weight amount and the acid value of the distillate and the residue obtained in each distillation step are shown in table 11.

**Table 11**

| | Sample sample | Weight (g) | Acid value (mg KOH/g) |
|---|---|---|---|
| 1st Distillation | 1st distillate | 113 | 197 |
| | 1st residue | 414 | 103 |
| 2nd Distillation | 2nd distillate | 196 | 186 |
| | 2nd residue | 217 | 27.7 |
| 3rd Distillation | 3rd distillate | 28 | 157 |
| | 3rd residue | 187 | 7.7 |

The results of the determination by a absorptiometry for the astaxanthin present in each distillate and residue obtained by the above distillation operations are shown in the following table 12.

**Table 12**

| Sample | Content of astaxanthin (%) |
|---|---|
| Raw material | 9.86 |
| After dehydration | 9.96 |
| 1st distillate | 0.01 |
| 1st residue | 12.6 |
| 2nd distillate | Trace |
| 2nd residue | 24.1 |
| 3rd distillate | Trace |
| 3rd residue | 27.11 |

Each ratio (%) of the free, monoester and diester forms of astaxanthin estimated by HPLC method is shown in table 13.

**Table 13**

| Sample Sample | Diester (%) | Monoester (%) | Free form (%) |
|---|---|---|---|
| Raw material | 11.82 | 83.75 | 4.43 |
| After dehydration | 12.02 | 83.87 | 4.10 |
| 1st distillate | 6.90 | 41.66 | 51.45 |
| 1st residue | 12.14 | 83.70 | 4.16 |
| 2nd distillate | 8.96 | 86.03 | 5.01 |
| 2nd residue | 12.23 | 83.73 | 4.04 |
| 3rd distillate | 34.35 | 63.91 | 1.74 |
| 3rd residue | 12.27 | 83.74 | 3.99 |

The content of astaxanthin could be concentrated to about 2.7 times by the above steps. The composition ratio of diester, monoester and free forms of astaxanthin was not changed by this treatment.

It can be seen from the above results that the degree of purification of astaxanthin was elevated by the distillation steps.

The crude astaxanthin has a peculiar odor. Although this odor component is unclear, it is assumed to be a low molecular compound. Since this odor component may be removed as the distillate by a molecular distillation, the residual astaxanthin fraction becomes no longer odor.

Astaxanthin has been said as being considerably unstable material and easily decomposed at an elevated temperature (Japanese Patent Application Laid-Open No. Hei-173058). As to the stability of astaxanthin against oxygen, heat and light, astaxanthin di- and monoesters has been said to be more stable than the free form of astaxanthin. According to the present invention, however, the decrease in the content of each free form, monoester and diester of astaxanthin was not recognized.

### Industrial Applicability

According to the present invention, a lipase is acted on a crude xanthophyll, which is obtained by extracting algae with a solvent, in the presence of water whereupon the contaminant (neutral lipids) present in said crude xanthophyll is selectively hydrolyzed, and the lipase-treated liquor is subjected to a water/ oil separation and free fatty acids is removed from the oil layer fractioned thereby the content of xanthophyll component could be elevated. Since the process of the present invention enable the hydrolysis reaction to proceed under a mild condition using a lipase, the decomposition of xanthophylls can be suppressed and the concentrated and purified material of xanthophyll (free form) and its ester form free of substantially peculiar odor can be obtained simply and conveniently in high yield without causing a change in each ratio of the free, monoester and diester forms of xanthophyll and therefore it is industrially extremely useful.

## Claims

1. A process for purifying a crude xanthophyll which is **characterized by** acting a lipase on the crude xanthophylls, which is obtained by extracting algae with a solvent, in the present of water under an acidic condition without adjusting the pH whereupon the contaminating neutral lipids (mono-, di- and triglycerides) are selectively hydrolyzed, subjecting the lipase -treated liquor to oil/water separation and removing free fatty acids from the oil layer fractioned thereby elevating the content of xanthophyll.

2. The process for purifying a crude xanthophyll as claimed in claim 1 wherein the crude xanthophyll which is obtained by extracting algae with a solvent is a crude astaxanthin, a crude zeaxanthin, a crude lutein, a crude tunaxanthin, a crude fucoxanthin, a crude violaxanthin, a crude neoxanthin or a crude capthaxanthin.

3. The process for purifying a crude xanthophyll as claimed in claim 1 wherein the crude xanthophyll is a crude astaxanthin and an acetone extract of Haematococcus algae.

4. The process for purifying a crude xanthophyll as claimed in claim 1 wherein the lipase is one belonging to genus *Candida.*
